# EUROPEAN PATENT APPLICATION

(11) **EP 1 445 331 A1**
(43) Date of publication of application: **11.08.2004**
(21) Application number: 04002726.0
(22) Date of filing: 06.02.2004
(51) Int. Cl.: C12Q 1/68

(54) **Molecular biological identification techniques for microorganism**

(30) Priority: 07.02.2003 JP 2003031488; 13.06.2003 JP 2003168941
(71) Applicant: Hiraishi, Akira, Toyohashi-shi, Aichi-ken (JP); NCIMB Japan Co., Ltd., Shizuoka-shi, Shizuoka-ken (JP)
(72) Inventor: Hiraishi, Akira, Toyohashi-shi, Aichi-ken (JP); NCIMB Japan Co., Ltd., Shizuoka-shi, Shizuoka-ken (JP)
(74) Representative: Glawe, Delfs, Moll & Partner

(57) **Abstract**

By using an ITS-DNA hybridisation technique, microorganisms are quickly differentiated and identified at the bacterial species level. By applying the principle to the DNA microarray, environmental microorganisms are detected and identified. By using ITS-DNA hybridisation and by applying the ITS-DNA hybridisation technique to the DNA microarray, differentiation at the bacterial species level or the relative species level becomes possible.

## Description

### TECHNICAL FIELD OF INVENTION

The present invention relates to a field of techniques including detection and identification of microorganisms, DNA-DNA hybridisation techniques, DNA macroarray techniques and DNA microarray techniques.

### PRIOR ART

Classification and identification of microorganisms, in particular bacteria, have so far been performed on the basis of nucleic acid molecular information as well as phenotypical information such as the cellular form, physiological and biochemical properties and chemical taxonomical properties. For classification and differentiation of high level taxons of genus or above, comparison of base sequences of small subunit rRNAs (or small subunit rDNAs, which are their genes) is effective. This molecular method, however, is difficult to use in differentiation and identification at the bacterial species level due to its low resolution. It is also difficult to identify the bacterial species from a single property or by a combination of phenotypes.

As the only method for differentiating bacterial species, the rate of genome DNA hybridisation between different bacterial strains (DNA-DNA homology) is used. The International Committee on Systematic Bacteriology recommended that a group of bacterial strains demonstrating a DNA-DNA hybridisation rate of 70% or higher should be acknowledged as the genetically same species (see Wayne et al. Int. J. Syst. Bacteriol, 37, 1987, pp. 463-464). This criterion is still generally accepted now.

The genome DNA-DNA hybridisation method has several technical defects. This method requires a relatively large amount of clean DNA samples. Accordingly, it takes labour and time to culture bacterial bodies, extract DNAs from cells, refine them and make specimens and probe samples. There are also other defects such as low reproducibility of hybridisation tests and unpredictable results because of contaminants, which are easily mixed in. This method also assumes that tests are conducted between pure bacteria strains that can be cultured, and as such it is not easily applicable for environmental samples, which can contain multiple microorganisms. This method is also difficult to apply in samples from which DNAs are hard to obtain.

As an alternative to the DNA-DNA homology for identifying the bacterial species, a molecular method that compares the base sequences of gyrB genes, for example, has been proposed in the Japanese unexamined patent application publication JP-A-2001-128679.

The method that determines the base sequences, however, not only takes time as in the case of the DNA-DNA hybridisation method, but it also requires selection of an appropriate amplifying primer for each bacterial species. This method is also difficult to apply in identification of multiple microorganisms existing in the environment.

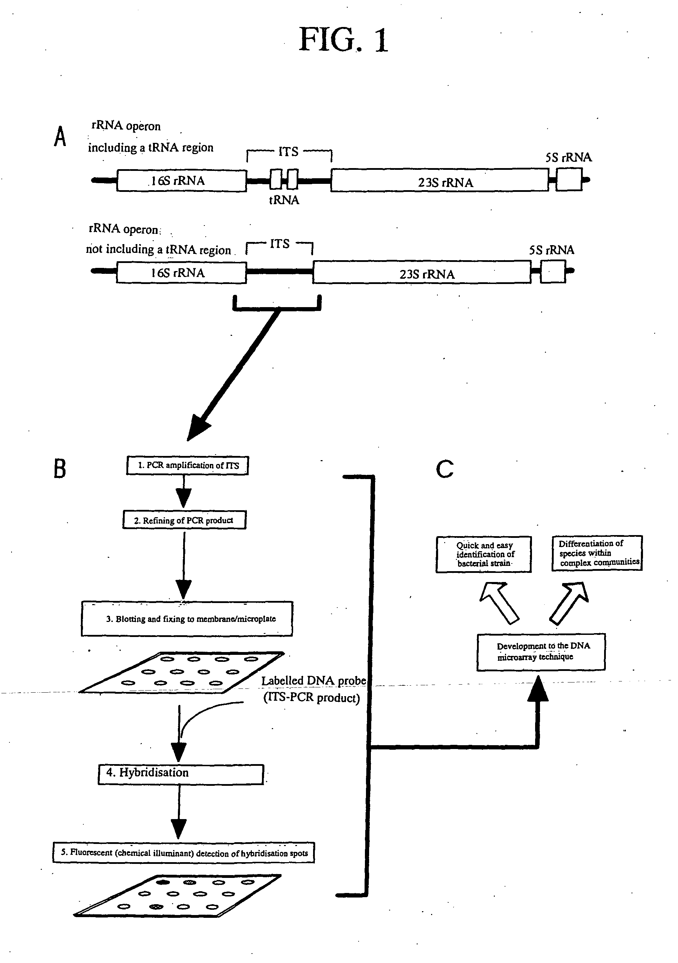

The ITS region (Fig. 1) existing in the rRNA gene operon includes a diversity of base sequences, and is therefore suggested to be effective in the development of probes for detecting "true" bacteria ("Eubacteria") (e.g., see Barry et al. PCR Methods Appl. 1, 1991, pp. 51-56). The information of its base sequences is now used in differentiation of microorganisms. Here, ITS is the abbreviation used in this document for "intergenic transcribed spacer" (i.e., ribosomal RNA spacer).

A method for detecting, separating and distinguishing Eubacteria taxons using an oligoprobe of sequences singular to the bacterial species originating in the ITS region is proposed e.g. in PCT (WO) H10-501976. This method using an oligoprobe of singular sequences requires the sequence information to design a probe. Qualitative reactions are used to differentiate the bacterial species.

So far there has been no report referring to hybridisation including all of the multiple-copy ITS region using a universal primer. Accordingly, there has been no specific evidence as to whether its ITS hybridisation rate is more effective in differentiating the bacterial species compared with the genome DNA hybridisation rate.
Also, there has been no report referring to the quantitative evaluation (relative strength) with regard to ITS hybridization rates.

There has been no report on any ITS hybridisation technique using the DNA microarray method.

Under and against the evidence and technical background described above, an objective of the present invention is to develop a molecular biological technique for differentiation and detection of the bacterial species of microorganisms having a quantitativeness and resolution that can replace the DNA-DNA hybridisation method and does not require any specific base sequence information, thereby performing identification of the bacterial species more speedily, easily and accurately than conventional techniques. A further objective of the present invention is to combine this technique with a DNA microarray thereby providing a new technique as a method for diagnosing environmental microorganisms that makes it possible to identify multiple bacterial species simultaneously and efficiently.

### SUMMARY OF THE INVENTION

In order to achieve the above objectives, the inventor procured typical species of gram negative bacteria and gram positive bacteria from a microorganism preservation organisation as well as newly separated bacteria from the natural environment, and analysed the ITS regions of a total of 1,500 strains.

First, the inventor extracted and refined genome DNAs from a test bacterial strain, and using these DNAs as moulds, performed PCR amplification on the ITS regions. After subcloning these PCR products (ITS-DNAs), base sequences were determined using a DNA sequencer. At the same time, a DNA-DNA hybridisation test was conducted. Analysis of the relationship between the base sequential differences among different bacterial strains and the DNA-DNA hybridisation rate has revealed that there was a high correlation between the two and that an ITS sequence homology of approximately 90% corresponded to a genome DNA-DNA hybridisation rate of 70%.

Based on these findings, hybridisation using ITS-DNAs was performed using the membrane filter method and on a microplate. From the strength of hybridisation signals obtained under certain hybridisation conditions, it was found that an ITS sequence hybridisation rate of 60 to 70% corresponded to a DNA-DNA hybridisation rate of 70%.
Based on these findings, the inventor has perfected the present invention relating to techniques for detecting and identifying microorganisms, their applications in the DNA macroarray technique and the DNA microarray technique, and an environmental diagnosis method.

In the present invention, a hybridisation method for DNAs corresponding to the ITS region (ITS-DNAs) is used in detection and identification of microorganisms. This makes it possible to differentiate at the bacterial species level and relative species level. This is due to the high correlation between the rate of hybridisation using genome DNAs and the rate of hybridisation using ITS-DNAs. The hybridisation method using genome DNAs is currently used as the only differentiation method for bacterial species, but it takes a lot of time and effort to test, and it is difficult to obtain reproducible results. Compared with the genome DNA, the ITS-DNA has an advantage in that it can be genetically amplified using PCR, etc., and it does not require a large amount of culture, making it possible to analyse easily and quickly. Because highly purified ITS-DNAs can be used for testing, reproducibility is high. Another advantage is that a universal primer can be used for PCR, making analyses easier than methods using other genes.

The ITS-DNA hybridisation is performed on a membrane filter or a microplate. The ITS-DNA hybridisation is also performed on a DNA microarray. By using these techniques, differentiation at the bacterial species level and relative species level becomes possible. When the ITS-DNA hybridisation technique is performed on a DNA microarray, a multitude of bacterial species can be identified efficiently, making this a convenient technique for detecting and identifying bacterial species included in unknown environmental samples.

The ITS-DNA hybridisation technique is also utilized in identification of strains of microorganisms. In this case, differentiation at the bacterial species level becomes possible by using a microarray on which a specific bacterial species (e.g., Bacillus, Pseudomonas, etc.) is fixed at the genus level or a microarray on which all known bacterial species are fixed. This technique is advantageous because it is quicker and easier than the hybridisation technique using genome DNAs.

The ITS-DNA hybridisation technique is also utilized in detection and identification of microorganisms in foodstuffs, foods and drinks. In this case, differentiation at the relative species level becomes possible by using a microarray on which a strain of food-derivative pure bacteria and food-poisoning-bacteria-derivative ITS-DNAs are fixed, or a microarray on which strains of all the known bacteria are fixed. When detecting microorganisms included in foodstuffs, foods and drinks, it is often required that bacterial species are detected quickly and at the bacterial strain level (e.g., in the detection of food-poisoning bacteria and contaminated bacteria). This technique is especially advantageous because the conditions of the relative species included in the samples can be known quickly from the identification results.

The ITS-DNA hybridisation technique is also utilized in detection and identification of microorganisms in living bodies such as clinical and enterobacteria. For example, in the case of human enterobacteria, differentiation at the relative bacterial level becomes possible by using a microarray on which a strain of gastrointestinal tract-derived pure bacteria is fixed or a microarray on which strains of all the known bacteria are fixed. In the human gastrointestinal tract, many bacteria exist. These bacteria form colonies called enteroflora while maintaining mutual relationships. The enteroflora changes according to a number of factors including meals and drugs taken everyday by a man. Bacteria that constitute enteroflora are also known to affect the man's health. If the constitution of the enteroflora can be monitored easily and quickly, it may be useful for the management of the man's health. A traditional method for discovering the constitution of the enteroflora has been to directly cultivate the enterobacteria included in the faeces. The ITS-DNA hybridisation method is particularly advantageous, even compared with the community analysis methods that are practised recently (e.g., DGGE, T-RFLP, FISH, etc.), because it can detect bacterial strains included in the sample at the relative species level and reveals the relative proportions of these strains.

The ITS-DNA hybridisation method is also used in detection and identification of microorganisms in other environments. In this case, differentiation at the relative species level becomes possible by using a microarray targeting a specific bacterial strain (e.g., thiobacillus ferrooxidans, actinomycetes, photosynthetic bacteria, etc.) or a microarray on which all the known species of bacteria are fixed. When detecting microorganisms in the environment, this method is advantageous in that it can detect bacteria that are difficult to culture and that it can identify a multitude of bacterial species simultaneously and efficiently.

The present invention will now be explained in detail in accordance with the preferred embodiments.

### BRIEF DESCRIPTION OF THE DRAWINGS

FIG. 1 is a schematic drawing showing an example of an ITS-DNA hybridisation technique of the present invention.
FIG. 2 is a graph showing the relationship between the ITS-DNA hybridisation rate according to the present invention and the genome DNA hybridisation rate.
FIG. 3 is a drawing showing a detection image of enterobacteria obtained by hybridisation with a DNA microarray using ITS-DNAs from a faeces sample.

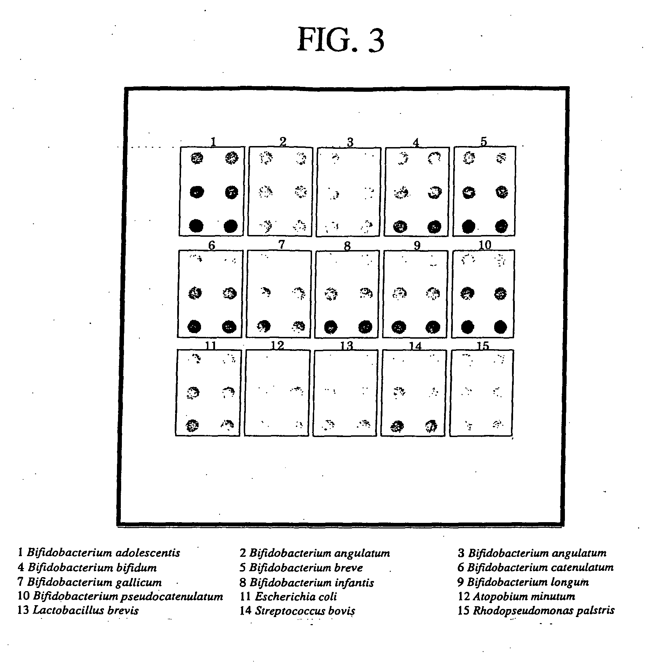

In Fig. 3 the numbers on the rectangles indicate sample numbers. In each rectangle there are three densities with two spots in each density.

### Preferred Embodiments of the Invention

In the case of bacteria, one to several copies of rRNA operons exist in a genome DNA. rRNA operons are arranged in the order of 16S, 23S and 5S rRNA from the upper stream and an ITS region exists between the 16S rDNA and the 23 S rDNA (Fig. 1A). The ITS consists of random base sequences free of ORF and rich in variety, but some copies have one or two preservation regions for coding tRNAs. The rRNA operons as a whole are relatively well preserved within the same bacterial species (sequential homology of 99% or higher), but among different species, sufficiently differentiable base sequential differences (approximately 90% or lower) can be obtained. The base sequential differences of the whole of the rRNA operons are detected using the ITS-DNA hybridisation.

An example of the ITS-DNA hybridisation will now be explained by reference to FIG. 1B. Because the 16S rDNA and the 23S rDNA include highly preservable base sequences, PCR primers targeting the preservation regions of the 3' end and the 5' end of each of the 16SrDNA and the 23SrDNA are used to PCR amplify ITS-DNAs. At this time a genome DNA extracted and refined from a bacterial body is used as a cast (FIG. 1B-1). For amplification, techniques other than PCR can also be used. The amplified ITS-DNAs are refined using electrophoresis, spin column or other methods (FIG. 1B-2). A certain amount of the refined target ITS-DNA samples is blotted on a membrane filter or a microplate and fixed (FIG. 1B-3). In the mean time, a probe is made by labelling an ITS-DNA using fluorescence, chemiluminescence or other labelling methods. Then hybridisation is performed between the membrane filter or the microplate on which the target ITS-DNA samples are fixed and the labelled probe (FIG. 1B-4). Spots of the hybridised DNAs are detected by fluorescence, chemiluminescence or other methods.

As PCR-amplification conditions for the ITS-DNA, the composition of the PCR reagent and the reactive conditions are shown in Table 1 and Table 2. The primer set may PCR amplify a sequence including ITS-DNAs or it may PCR amplify only a portion of ITS-DNAs. 1541f (sequence number: 1) and LS23r (sequence number: 2) are cited as examples. Other primer sets capable of PCR amplifying ITS-DNAs can also be used. After a PCR reaction, 2µl of the amplified product is confirmed using 1.5% agarose gel electrophoresis.

**Table 1**

| Per one reaction | |
|---|---|
| Sterilized water | 37.75µl |
| 10 x PCR buffer | 5.0 µl |
| dNTPs (2mM each) | 5.0 µl (final density 0.2 mM) |
| 20 µM 1512fprimer (sequence number: 1) | 0.5 µl (final density 0.2 µM) |
| 20 µM LS23r primer (sequence number: 2) | 0.5 µl (final density: 0.2 µM) |
| Taq DNA polymerase (5 units/µl)^{*2} | 0.25 µL (1.25 units) |
| Genome DANN | 1 µl (10ng) |

| | |
|---|---|
| *2: HotSarTaq Polymerase by QIAGEN (Cat #203203) is used. | |

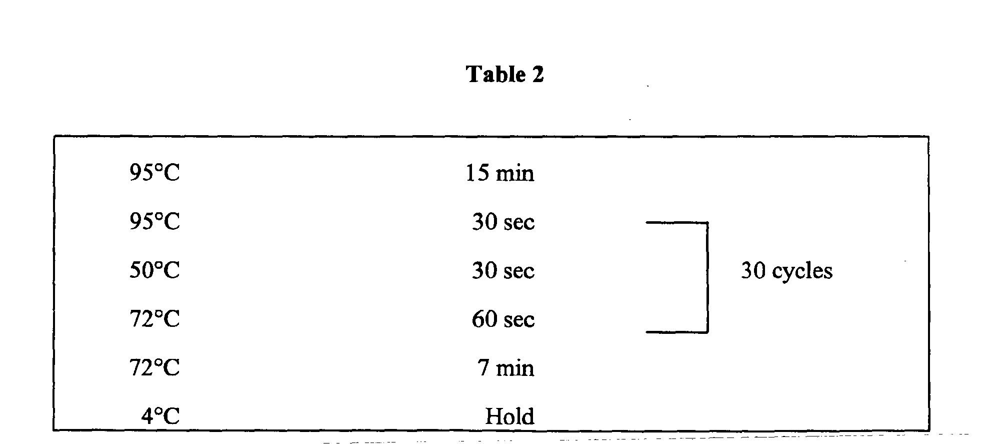

(Sequence number: 1)
Sequence length: 17
Sequence type: DNA
Number of strands: 1
Topology: Straight strand
Sequence category: Other nucleic acids, synthetic DNA
Origin

Biological name: Escherichia coli
Sequence
ggctggatca cctcctt
(Sequence number 2)
Sequence length: 17
Sequence type: DNA
Number of strands: 1
Topology: Straight strand
Sequence category: Other nucleic acids, synthetic DNA
Origin
Biological name: Escherichia coli
Sequence
tgcccaaggc atccacc

PCR amplified products were refined using QIA quick™ PCR Purification Kit (QIAGEN). Amplification of the refined amplified products was confirmed by electrophoresis in a 1.5% agarose gel and ethidium bromide staining.

An example in which hybridisation is performed using a membrane filter is shown below.

Fixing of ITS-DNAs onto the membrane filter (Hybond+, Amersham, Cat#RPN2020B) is conducted under the following conditions.
(1) After soaking a membrane filter cut to an appropriate size in distilled water, submerge the membrane filter in 2 × SCC for at least 10 minutes and dry it in a desiccator for one day.
(2) Prepare 10 µl of 0.2 µM ITS-DNAs and subject it to heat denaturation at 100°C for 5 minutes.
(3) Rapidly cool the denatured DNAs on ice, and add half the amount of 20 x SSC to the ITS-DNAs to dilute them just before spotting.
(4) Place the membrane filter on filter paper and spot the ITS-DNAs for 2 µl while making sure that the tip of the pipette does not touch the membrane.
(5) After blotting excess moisture on the membrane filter, dry the membrane filter at 80°C for 10 minutes.
(6) Fix the ITS-DNAs on the membrane filter using ultraviolet radiation.

When performing hybridisation using a membrane filter, ITS-DNAs are labelled under the following conditions. For labelling of probes, heat-resistant alkaline phosphatase (AlkPhos Direct Labelling System System Amersham, Cat#RPN3680) is used, but other labels can also be used.
(1) Add 40 µl of sterilized water included in the kit to 10 µl of Cross-linking solution and dilute it.
(2) Dilute the ITS-DNA to be labelled with sterilized water so that the density will be 100 ng/µl.
(3) Put 10 µl of the ITS-DNA to be labelled into a microtube and subject it to heat denaturation at 100°C for 5 minutes.
(4) Rapidly cool the denatured ITS-DNA on ice, and lightly centrifuge it in a desktop centrifugal separator.
(5) Add 10 µl of reaction buffer to the ITS-DNA made in step (4). Let reaction occur on ice and perform gentle mixing.
(6) Add 2 µl of labelling reagent. Let reaction occur on ice and perform gentle mixing.
(7) Add 10 µl of the diluted Cross-linking solution. After mixing it completely, lightly centrifuge the solution in the desktop centrifugal separator.
(8) Incubate at 37°C for 30 minutes.

Hybridisation using a membrane filter is performed under the following conditions.
(1) Evenly dampen a membrane filter on which ITS-DNAs are fixed with 2 × SCC. Blot excess moisture on the membrane filter using filter paper.
(2) Add the membrane filter to a hybridisation buffer (AlkPhos Direct Labelling System Amersham, Cat#RPN3680) that is pre-heated to 70°C by a hybridisation oven.
(3) Add a PCR amplified product that is in the rDNA, from which ITS included in the PCR primer or PCR product has been removed for the purpose of masking, to the hybridisation buffer and hybridise it at 70°C for 30 minutes.
(4) Add all the ITS-DNAs labelled on the buffer used in the prehybridisation. (At this time take a small amount of buffer and mix it in the microtube.)
(5) Incubate at 70°C for 2 hours. (Watch that the solution does not evaporate.)
(6) Soak the membrane filter in the primary rinse buffer that is pre-heated, and rinse it at 50°C for 10 minutes by gently shaking it. Repeat this step twice.
(7) Put 250 ml of secondary rinse buffer into a new container, soak the membrane filter in it and rinse it at room temperature for 5 minutes. Repeat this step twice.

When performing hybridisation using a membrane filter, signals are detected under the following conditions. Chemical illumination (CDP Star, Amersham, Cat#RPN3682) is used for detection.
(1) Remove excess secondary rinse buffer from the membrane filter, and put the membrane filter on a Saran film with its blotted surface facing up.
(2) Add 800 µl of CDP-Star Detection reagent to the membrane filter and leave it for 5 minutes.
(3) Blot excess buffer with filter paper. Wrap the membrane filter with a Saran film such that there are no air bubbles beneath the film and place it on the film cassette with the blotted surface facing up. Expose the film for 2 hours in a dark room.

The ITS-DNA hybridisation according to the present invention can be used for bacterial species level identification of bacterial strains purely cultured by the DNA macroarray method using a membrane filter or a microplate. In addition, the ITS-DNA hybridisation of the present invention can also be applied for use with DNA microarrays having the object of detecting relative species from samples containing a wide variety of microorganisms (FIG. 1C).

An example of hybridisation using a hollow thread sequence DNA microarray is shown below.

Fix ITS-DNAs onto the DNA microarray under the following conditions. Fasten one strand of the ITS-DNA to the hollow thread. Set the density of the ITS-DNA in three stages (e.g., 0.06, 0.04 and 0.02 µM) and spot at three points. To fix the spots, other methods can also be used.

When performing hybridisation using a DNA microarray, ITS-DNAs are labelled by adding a PCR primer labelled with fluorescent dye Cy5 to the PCR reagent (counter strand of the ITS-DNA fixed on the DNA microarray). Other labelling methods can also be used.

Hybridisation using a DNA microarray is performed under the following conditions.
(1) Soak the DNA microarray in sterilized water at 65°C for 6 hours.
(2) Prepare 25 µl of 20 x SSC, 10 µl of 2% SDC and 15 µl of sterilized water, and keep them at the temperature of 37°C.
(3) Heat-denature 50 µl of the labelled 100 ng/µl ITS-DNA at 94°C for 2 minutes. Then rapidly cool it on ice for 1 minute.
(4) Place the DNA microarray in the hybridisation chamber. Pour 100 µl of the prepared solution and perform hybridisation at 65°C for 16 hours.
(5) Rinse the DNA microarray by putting it into 10 ml of 0.05 x SSC, which has been kept at 50°C in a centrifuge tube, and leaving it still for 40 minutes.
(6) Rinse the DNA microarray by putting it into 10 ml of fresh 0.05 x SSC, which has been kept at 50°C in a centrifuge tube, and leaving it still for 20 minutes.
(7) Leave the DNA microarray at 4°C for 5 minutes.

When performing hybridisation using a DNA microarray, a fluorescent reader is used for detection.

The DNA macroarray technique using ITS-DNA hybridisation and the DNA microarray technique can be used for simple and quick identification of pure bacterial strains, as well as for detection and identification of the bacterial species of microorganism in a diversity of environments. The environmental samples that can be tested include water, soil, air, sewage and waste water treatment systems, as well as foods, human and animal samples (e.g., faeces, saliva, etc.) and clinical materials.

The present invention will now be explained in detail using examples.

### EXAMPLES

### (Example 1)

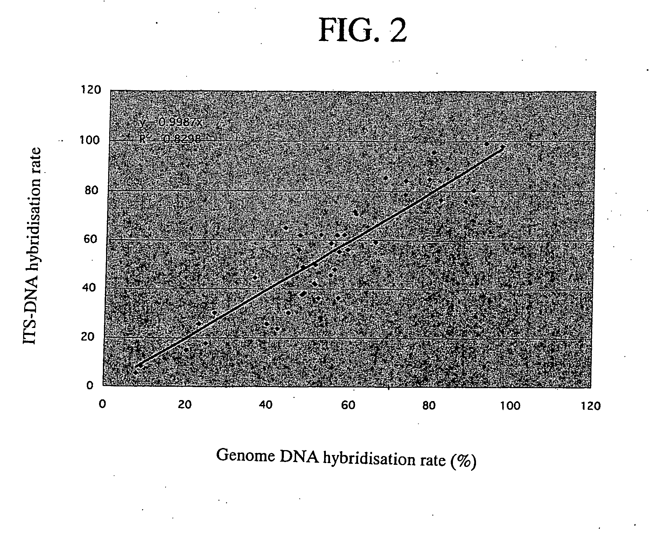

Using bacterial strains belonging to the Rhodoplanes genus, which is a photosynthetic bacterium, an ITS-DNA hybridisation test and a genome DNA hybridisation test were conducted for comparison. FIG. 2 shows the ITS-DNA hybridisation rate as contrasted with the genome DNA hybridisation rate. A strong primary functional correlation of X = Y was seen between the two. This result indicates that 70% of the genome DNA hybridisation rate corresponds to approximately 70% of the ITS-DNA hybridisation rate.

### (Example 2)

DNAs were extracted from faeces samples. ITS-DNAs that were amplified and labelled from the extracted DNAs were used for hybridisation with a DNA microarray on which ITS-DNAs of enterobacteria of the base strain were fixed. Detection was performed at the enterobacterial species level. FIG. 3 shows a detection image of enterobacteria obtained by hybridisation of the faeces samples ITS-DNAs with a DNA microarray. The result shows that a DNA microarray using ITS-DNA is effective in the detection and identification of the bacterial species of microorganisms in a variety of samples.

### EFFECTS OF THE INVENTION

The invention described in claim 1 has the effect of making possible differentiation at the bacterial species or relative species level. The ITS-DNA has an advantage over the genome DNA in that the ITS-DNA can be genetically amplified by PCR, etc., does not require a large amount of culture and can be easily and quickly analysed. Another advantage of the ITS-DNA is that ITS-DNAs of high purity can be used for testing so that reproducibility is relatively high. Furthermore, because a universal primer can be used for PCR, analyses can be conducted more easily than the methods using other genes.

The invention described in claim 2 or 3 has the effect of making possible differentiation at the bacterial species or relative species level.

The invention described in claim 4 has the effect of making possible differentiation at the bacterial species or relative species level. The invention is also advantageous in detecting and identifying the bacterial species included in unknown environmental samples because many bacterial species can be efficiently identified.

The invention described in claim 5 has the effect of making possible differentiation at the bacterial species level by using a microarray on which specific bacterial species are fixed at the genus level or a microarray on which all the known bacterial species are fixed. The invention is also advantageous in that it is quicker and easier compared with the hybridisation method using genome DNAs.

The invention described in claim 6 has the effect of making possible differentiation at the relative species level by using a microarray on which pure bacterial strains derived from foodstuffs, foods and drinks and ITS-DNAs derived from food-poisoning bacteria are fixed or a microarray on which all known bacterial strains are fixed. The invention is particularly advantageous because the property of the relative bacteria included in the sample becomes immediately available from the identification result.

The invention described in claim 7 has the effect of making possible differentiation at the relative bacterial species level by using a microarray on which pure bacterial strains derived from organisms including clinical and enterobacteria are fixed or a microarray on which all the known bacterial species are fixed. Compared with the community analysis methods that are practised recently, the invention is advantageous in that it makes possible detection of bacterial strains included in the sample at the relative species level and observation of the relative ratios of each bacterial strain.

The invention described in claim 8 has the effect of making it possible differentiation at the relative bacterial species level by using a microarray targeting specific bacterial species or a microarray on which all the known bacterial species are fixed. The invention is also advantageous in detecting microorganisms in the environment because bacteria that are difficult to culture can also be detected and many bacterial species can be concurrently and efficiently identified.

## Claims

1. A molecular biological identification technique for microorganisms using hybridisation of DNA corresponding to the ITS region ("ITS-DNA").

2. A molecular biological identification technique for microorganisms, **characterized in that** the ITS-DNA hybridisation as claimed in claim 1 is performed on a membrane filter.

3. A molecular biological identification technique for microorganisms, **characterized in that** the ITS-DNA hybridisation as claimed in claim 1 is performed on a microplate.

4. A molecular biological identification technique for microorganisms, **characterized in that** the ITS-DNA hybridisation as claimed in claim 1 is performed on a DNA microarray.

5. A molecular biological identification technique for microorganisms **characterized in that** the technique described in any of claims 2, 3 and 4 is used in identification of strains of microorganism.

6. A molecular biological identification technique for microorganisms **characterized in that** the technique described in any of claims 2, 3 and 4 is used in detection and identification of microorganisms in foodstuffs, foods and drinks.

7. A molecular biological identification technique for microorganisms **characterized in that** the technique described in any of claims 2, 3 and 4 is used in detection and identification of microorganisms in a living body, including clinical and enterobacteria.

8. A molecular biological identification technique for microorganisms **characterized in that** the technique described in any of claims 2, 3 and 4 is used in detection and identification of microorganisms in other environments.


SEQUENCE LISTING

<110> NCIMB Japan Co., Ltd.

Hiraishi, Akira

<120> Molecular biological identification techniques for microorganisms

<130> H15P1027

<150> JP 2003-031488

<151> 2003-02-07

<150> JP 2003-168941

<151> 2003-06-13

<160> 2

<170> PatentIn version 3.1

<210> 1

<211> 17

<212> DNA

<213> Escherichia coli

<400> 1

ggctggatca cctcctt 17

<210> 2

<211> 17

<212> DNA

<213> Escherichia coli

<400> 2

tgcccaaggc atccacc 17